(19) Europäisches Patentamt
European Patent Office
Office européen des brevets

(11) **EP 3 328 446 B1**

(12) **EUROPÄISCHE PATENTSCHRIFT**

(45) Veröffentlichungstag und Bekanntmachung des Hinweises auf die Patenterteilung:
**18.09.2019 Patentblatt 2019/38**

(21) Anmeldenummer: **16741929.0**

(22) Anmeldetag: **20.07.2016**

(51) Int Cl.:
*A61L 2/26* *(2006.01)*        *A61B 1/00* *(2006.01)*
*G06M 3/12* *(2006.01)*

(86) Internationale Anmeldenummer:
**PCT/EP2016/067332**

(87) Internationale Veröffentlichungsnummer:
**WO 2017/016958 (02.02.2017 Gazette 2017/05)**

(54) **VORRICHTUNG ZUM ZÄHLEN VON STERILISATIONSZYKLEN**

DEVICE FOR COUNTING STERILISATION CYCLES

DISPOSITIF DE COMPTAGE DE CYCLES DE STÉRILISATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.07.2015 DE 102015112205**

(43) Veröffentlichungstag der Anmeldung:
**06.06.2018 Patentblatt 2018/23**

(73) Patentinhaber: **Aesculap AG**
**78532 Tuttlingen (DE)**

(72) Erfinder: **WEISSHAUPT, Dieter**
**78194 Immendingen (DE)**

(74) Vertreter: **Winter, Brandl, Fürniss, Hübner, Röss, Kaiser, Polte - Partnerschaft mbB Patent- und Rechtsanwaltskanzlei Alois-Steinecker-Strasse 22 85354 Freising (DE)**

(56) Entgegenhaltungen:
**EP-A1- 0 979 658        DE-A1-102007 039 088
US-A- 5 359 993        US-A1- 2014 000 506**

EP 3 328 446 B1

**Beschreibung**

Die vorliegende Erfindung betrifft eine Vorrichtung zum Zählen von Sterilisationszyklen bei der Sterilisierung medizinischer Instrumente und Vorrichtungen.

Im klinischen Alltag ist es insbesondere aus Gründen der Qualitätssicherung oftmals erforderlich, die Anzahl von Sterilisationszyklen zu erfassen, denen medizinische Instrumente, Instrumentensets oder Sterilisationscontainer unterworfen werden. Bislang erfolgt eine derartige Erfassung von Hand und durch das jeweilige Fachpersonal, beispielsweise durch Führen von Strichlisten oder Tabellen. Dass eine derartige Überwachung der Anzahl von Sterilisationszyklen eines Instruments mit Unsicherheiten behaftet und für eine Überprüfung ungeeignet ist, liegt auf der Hand. Es ist von Nachteil, dass derzeit keine Möglichkeit besteht, eine Anzahl von Sterilisationszyklen, denen ein bestimmtes Instrument unterzogen wird, verlässlich, nachprüfbar und automatisiert zu erfassen.

Aus der Druckschrift EP 0 979 658 A1 ist eine Vorrichtung zum Zählen von Sterilisationszyklen bekannt, die ein Zählwerk un deine Betätigungseinrichtung aufweist zur temperaturabhängigen Batätigung des Zählers, sowie eine Sperreinrichtung. Erst nach Überschreiten einer ersten Temperatur und Unterschreiten einer zweiten Temperaturist die Sperreinrichtung entsperrt und die Betätigungseinrichtung kann den Zähler betätigen.Die Druckschriften DE 10 2007 039088 A1, US 5 359 993 A und US 2014/000506 A1 offenbaren weitere Vorrichtung zum Zählen von Sterilisationszyklen aus denen die Verwendung temperatursensibler Materialien zur Betätigung eines Sterilisationszyklenzählers bekannt ist.

Vor diesem Hintergrund liegt der vorliegenden Erfindung die Aufgabe zugrunde, eine Möglichkeit und insbesondere eine Vorrichtung zu schaffen, mit der die Anzahl von Sterilisationszyklen einfach, sicher, fehlerfrei, automatisch und überprüfbar erfasst und festgehalten werden kann. Dabei ist insbesondere sicherzustellen, dass auch ein Sterilisationszyklus mit schwankendem Druck- und/oder Temperaturverlauf nur als ein Zyklus erfasst wird und falsche Mehrfacherfassungen infolge solcher Druck- und/oder Temperaturverläufe sicher vermieden werden.

Diese Aufgabe wird nach der vorliegenden Erfindung gelöst durch eine Vorrichtung zum Zählen von Sterilisationszyklen bei der Sterilisierung medizinischer Instrumente und Vorrichtungen, aufweisend ein Zählwerk zum Aufzeichnen und Wiedergeben einer Anzahl der Sterilisationszyklen, eine Betätigungseinrichtung zur sterilisationsparameterabhängigen Betätigung des Zählwerks, sowie eine Sperreinrichtung zum sterilisationstemperaturabhängigen Sperren des Zählwerks, wobei die Betätigungseinrichtung bei Überschreiten eines Schwellsterilisationsdrucks das Zählwerk betätigt und die Sperreinrichtung das Zählwerk nach einer erfolgten Betätigung und/oder nach Überschreiten einer ersten Schwellsterilisationstemperatur, sperrt und nach Unterschreiten einer zweiten Schwellsterilisationstemperatur, entsperrt.

Durch die Erfindung wird unter Ausnutzung gegebener Parameter während eines Sterilisationsprozesses das Zählwerk automatisch betätigt und gesperrt. Durch die Sperreinrichtung kann eine Unterbrechung der Betätigung des Zählwerks mittels der Betätigungseinrichtung erfolgen, so dass sichergestellt ist, dass das Zählwerk pro Sterilisationszyklus nur einmal betätigt wird und die Anzahl von Sterilisationszyklen korrekt erfasst wird, unabhängig vom jeweiligen Verlauf. Die Betätigung kann beispielsweise abhängig vom Sterilisationsdruck erfolgen, wobei das Sperren/Entsperren abhängig von der Sterilisationstemperatur erfolgt. Alternativ kann die Betätigung abhängig von der Sterilisationstemperatur erfolgen, wobei das Sperren/Entsperren abhängig vom Sterilisationsdruck erfolgt.

Das Zählwerk ist vorzugsweise rein mechanisch ausgebildet. Es kann allerdings stattdessen ein elektronisch arbeitendes Zählwerk verwendet werden. In beiden Fällen erfolgt seine Betätigung mittels der Betätigungseinrichtung in mechanischer Weise. Ein rein mechanisches Zählwerk hat den Vorteil, dass es ohne Probleme sterilisiert und aufbereitet werden kann. Das Zählwerk kann an den weiteren Bestandteilen der erfindungsgemäßen Vorrichtung angeordnet oder montiert sein, insbesondere austauschbar. Es kann alternativ in die Vorrichtung integriert sein, zum Beispiel in einem gemeinsamen Gehäuse aufgenommen sein oder ein solches ausbilden.

Die Betätigungseinrichtung bewirkt eine Betätigung des Zählwerks. Infolge einer Betätigung zählt dieses einen Schritt weiter. Beispielsweise kann die Betätigungseinrichtung relativ zum Zählwerk eine Positionsänderungen ausführen und so ein Verstellen des Zählwerks, also einen Zählschritt bewirken. Die Betätigungseinrichtung arbeitet nach der Erfindung zum Beispiel sterilisationsdruckabhängig. Das bedeutet, dass sie in Abhängigkeit vom bei einem Sterilisationszyklus vorliegenden Druck, zum Beispiel dem Druck in einer Sterilisationskammer, der auf die erfindungsgemäße Vorrichtung wirkt, eine Betätigung des Zählwerks bewirkt. In Abhängigkeit des Drucks umfasst insbesondere eine Betätigung des Zählwerks bei Überschreiten eines Schwellenwerts (Schwellsterilisationsdruck), der vorzugsweise durch einen Nutzer der Vorrichtung einstellbar sein kann, z.B. durch Vorsehen einstellbarer Vorspannfedern oder Ähnlichem. Man kann also sagen, dass die Betätigungseinrichtung einen Zählschritt des Zählwerks bewirkt, wenn im Rahmen eines Sterilisationsprozesses ein bestimmter Sterilisationsdruck erreicht wurde.

Die Sperreinrichtung ist derart ausgebildet, dass sie nach einer Betätigung des Zählwerks mittels der Betätigungseinrichtung das Zählwerk in Abhängigkeit von Parametern des Sterilisierungsprozesses für abermalige Betätigungen vor Ende der Sterilisation sperrt. Erfindungsgemäß funktioniert die Sperreinrichtung zum Beispiel in Abhängigkeit der jeweils vorliegenden Sterilisationstemperatur, der die Vorrichtung nach der Erfindung ausgesetzt ist. Die Sperreinrichtung kann mechanisch in einer Stellung relativ zur Betätigungseinrichtung und/oder dem Zählwerk gehalten sein, in der eine Betätigung des Letzteren durch die Betätigungseinrichtung möglich ist. Infolge der Betätigung kann diese mechanische Blockade der Sperreinrichtung aufgehoben werden, so dass sich diese in Abhängigkeit der auf sie wirkenden Steuerungsparameter, insbesondere in Abhängigkeit von der Temperatur, aus ihrer ursprünglichen Freigabestellung in eine Sperrstellung bewegen kann. Alternativ oder zusätzlich kann die Sperreinrichtung durch Erreichen eines Schwellparameters, z.B. Schwellsterilisationstemperatur, deblockiert werden.

In der Sperrstellung ist eine weitere Betätigung des Zählwerks durch die Betätigungseinrichtung nicht möglich. Die Sperreinrichtung kann zum Beispiel die Betätigungseinrichtung nach Art eines Riegels mechanisch verriegeln, so dass eine zur Betätigung des Zählwerks erforderliche Relativbewegung nicht möglich ist. Die die Sperreinrichtung steuernden genannten ersten und zweiten Schwellsterilisationstemperaturen können identisch oder verschieden sein. Bei einer Ausführungsform der Erfindung kann also für einen Zählschritt des Zählwerkes erforderlich sein, dass die Schwellwerte beider Zustandsgrößen Temperatur und Druck in der Sterilisationskammer vorhanden sind, also die jeweils erforderlichen Schwellwerte annehmen oder über- oder unterschreiten, andernfalls kommt es zu keiner Betätigung des Zählwerks.

Die erfindungsgemäße Vorrichtung kann eigenständig verwendet werden, beispielsweise neben zu sterilisierenden Instrumenten in eine Sterilisationskammer oder einen Sterilisationsbehälter eingebracht werden. Sie kann aber auch in zu sterilisierende Instrumente oder Sterilisationsgerätschaften (Behälter) integriert sein. Insgesamt schafft die Erfindung eine einfache, robuste und kostengünstige rein mechanische Möglichkeit, die Anzahl von Sterilisationszyklen zu erfassen und zu zählen.

Mit der Erfindung können insbesondere die im Folgenden aufgelisteten Vorteile erzielt werden: Nach einmaliger Betätigung des Zählwerks erfolgt automatisch eine Entkopplung der Betätigungseinrichtung vom Zählwerk, so dass Fehlbetätigungen sicher vermieden werden können. Das Zählwerk kann bedenkenlos in einer Spülmaschine aufbereitet werden, ohne dass es dabei fehlerhaft weitere Zählschritte aufzeichnet. Das Zählwerk kann eigenständig verwendet werden und in oder auf einem Siebkorb liegen. Das Zählwerk kann in eine Frontblende eines Sterilisierungscontainers integriert sein. Das Zählwerk kann einfach genullt/resettet werden.

Vorteilhafte Ausführungsformen der Erfindung sind in den Unteransprüchen beansprucht und werden nachfolgend näher erläutert.

Bei einer Ausführungsform der Erfindung weist die Betätigungseinrichtung eine Kolben-Zylinder-Einheit mit einer vom Sterilisationsdruck beaufschlagbaren Kammer auf. Der Kolben und der Zylinder können in Abhängigkeit vom Sterilisationsdruck zur Betätigung des Zählwerks zueinander relativpositionierbar sein. Neben der mit dem Sterilisationsdruck beaufschlagten Kammer kann die Kolben-Zylinder-Einheit eine Druckkammer aufweisen. In dieser kann ein vorzugsweise durch einen Nutzer einstellbarer Nenndruck als dem Sterilisationsdruck entgegenwirkenden Gegendruck vorliegen. Durch Einstellen des Gegendrucks in der Druckkammer kann der jeweilige Ansprechdruck (Schwellwert des Sterilisationsdrucks) eingestellt werden.

Eine weitere Ausführungsform der Erfindung ist dadurch gekennzeichnet, dass die Sperreinrichtung eine gegenüber der Kolben-Zylinder-Einheit bewegbare, insbesondere rotierbare Scheibe, mit einem Durchlass für das Betätigungselement aufweist. Sind Durchlass und Betätigungseinrichtung zueinander ausgerichtet, ist eine Betätigung des Zählwerks möglich. In anderen Stellungen der Scheibe relativ zur Betätigungseinrichtung ist die letztere gesperrt, so dass eine Betätigung nicht möglich ist. Die Scheibe, die auch als Steuerscheibe bezeichnet werden kann, wirkt dann nach Art einer mechanischen Sperre auf die Betätigungseinrichtung. Vorzugsweise ist die Scheibe gegenüber dem Betätigungselement drehpositionierbar.

Die Sperreinrichtung kann insbesondere ein Bimetallelement aufweisen. Dieses kann sich in Abhängigkeit der Sterilisationstemperatur zwischen einer ersten Gestalt und einer zweiten Gestalt verformen. Die Relativposition der Scheibe zur Betätigungseinrichtung kann auf diese Weise durch Formänderung des Bimetallelements bestimmt werden. Die Scheibe kann also mit dem Bimetallelement bewegungsgekoppelt sein. Sie kann insbesondere bei in der ersten Gestalt befindlichem Bimetallelement eine erste Stellung und bei in der zweiten Gestalt befindlichem Bimetallelement eine zweite Stellung einnehmen. In der ersten Gestalt kann das Zählwerk von der Betätigungseinrichtung entkoppelt sein, befindet sich die Scheibe demnach in ihrer Sperr- oder Verriegelungsstellung. In der zweiten Gestalt kann das Zählwerk mit der Betätigungseinrichtung gekoppelt sein, befindet sich die Scheibe also in ihrer Freigabestellung. Insbesondere kann das Bimetallelement derart ausgebildet sein, dass es sich bei Überschreiten einer ersten Schwellsterilisationstemperatur aus seiner ersten Gestalt in seine zweite Gestalt verformt. Es kann sich bei Unterschreiten einer zweiten Schwellsterilisationstemperatur aus seiner zweiten Gestalt zurück in seine erste Gestalt verformen. Die erste und die zweite Schwellsterilisationstemperatur können gleich oder verschieden sein.

Des Weiteren kann die Betätigungseinrichtung nach einer Ausführungsform der Erfindung einen Zapfen zur Betätigung des Zählwerks aufweisen. Die Steuerungsscheibe kann mit einem Durchlass für einen solchen Zapfen versehen sein. Bei in der ersten Stellung befindlicher Steuerungsscheibe ist dieser vorzugsweise in Überdeckung mit dem Zapfen positioniert und eine Betätigung des Zählwerks ist möglich. Bei in der zweiten Stellung befindlicher Scheibe ist der Zapfen gesperrt, kann nicht durch den Durchlass hindurchgreifen, und eine Betätigung des Zählwerks ist nicht möglich. Bei einer Form der Erfindung ist der Zapfen als Kolbenstange ausgebildet und mittelbar, vorzugsweise unmittelbar mit dem Kolben der Kolben-Zylinder-Einheit verbunden. In ähnlicher Weise kann das Zählwerk einen Stift oder Zapfen aufweisen. Die Betätigungseinrichtung kann diesen mittelbar oder unmittelbar betätigen. Der Zählwerkszapfen kann in eine Ausnehmung der Steuerscheibe eingreifen und diese so in deren Freigabestellung blockieren. Greifen Zählwerkszapfen und Betätigungseinrichtung in die gleiche Ausnehmung der Steuerscheibe ein, kann diese im Rahmen einer Betätigung des Zählwerks besonders einfach deblockiert werden.

Zwischen der Betätigungseinrichtung und dem Zählwerk kann in dem Durchlass der Steuerungsscheibe ein Kraftübertragungselement, vorzugsweise eine Rolle oder eine Kugel, angeordnet sein. Dies ist insbesondere im Fall einer Betätigungseinrichtung mit Zapfen/Kolbenstange von Vorteil. Das Kraftübertragungselement ermöglicht in vorteilhafter Weise eine zur Betätigung des Zählwerks erforderliche Betätigungskraft, entkoppelt jedoch andere Kräfte und Momente, so dass eine unerwünschte Fehlbelastung des Zählwerks verhindert werden kann. Bei einer Ausführungsform mit Druckkammer wird diese nicht blockiert, was bedeutet, dass sich der Kolben frei im Zylinder bewegen kann. Dies schont das gesamte System.

Bei einer Ausführungsform der Erfindung kann der Durchlass in der Steuerungsscheibe einen ersten Abschnitt mit einer ersten Weite und einen zweiten Abschnitt mit einer gegenüber dem ersten Abschnitt kleineren Weite aufweisen. Insbesondere kann dabei das Kraftübertragungselement in dem ersten Abschnitt angeordnet sein. Die Weite des Kraftübertragungselements ist vorzugsweise größer als die Weite des zweiten Abschnitts. Nach einer Ausführungsform kann der Zapfen eine Weite aufweisen, die größer als die Weite des ersten Abschnitts ist. Er kann an seinem dem Kraftübertragungselement zugewandten Endabschnitt eine verringerte Weite aufweisen, die kleiner als die Weite des zweiten Abschnitts ist.

Vorzugsweise ist die Steuerungsscheibe rotierbar auf einem Führungsbolzen aufgenommen. Sie kann insbesondere eine zentrale Öffnung, vorzugsweise eine Durchgangsöffnung aufweisen, in der der Führungsbolzen relativ zur Scheibe rotierbar aufgenommen ist. Das Bimetallelement ist vorzugsweise mit einem Endbereich an dem Führungsbolzen angeordnet und kann insbesondere von diesem in radialer Richtung abstehen. Mit seinem diesem Endbereich gegenüberliegenden Endbereich ist es dann mit der Steuerungsscheibe verbunden.

Vorzugsweise weist der Zylinder ein Zylindergehäuse auf, in dem der Kolben axialpositionierbar aufgenommen ist. Des Weiteren ist darin außerdem die Steuerungsscheibe rotierbar aufgenommen. Endseitig an oder in dem Zylindergehäuse ist dann das Zählwerk aufgenommen, gegenüber dem Zylindergehäuse nicht relativpositionierbar. Das Zählwerk kann nach der Erfindung in beliebiger Weise ausgebildet sein, zum Beispiel mechanisch oder digital. Bevorzugt ist aufgrund seiner Robustheit jedoch ein mechanisches Zählwerk.

Zusammenfassend kann man auch sagen, dass mit der Erfindung ein mechanisches Zählwerk mit einer hermetisch abgeschlossenen Druckkammer zur Betätigung desselben kombiniert wird. Die Kammer ist mit einem geeigneten Gas oder Luftgemisch gefüllt. Bei einem Überdruck und bei einem Unterdruck in der Sterilisationskammer dehnt sich das Gas aus bzw. zieht sich zusammen. Um diese Zustände des Gases zu nutzen, kann die Kammer mit einer elastischen Membrane versehen sein. Bei einer Ausführungsform der Erfindung kann die Paarung Zylinder/Kolben so genau arbeiten, dass sie luft- bzw. gasdicht ist. Die Membrane bzw. der Kolben bewirkt einen Ausgleich von Druckänderungen und unterliegt infolge dessen zwangsläufig Positionsänderungen, legt zum Beispiel eine bestimmte Wegstrecke zurück. Diese Positionsänderung wird genutzt, um das Zählwerk zu schalten oder zu betätigen.

Während eines Sterilisationsprozesses können sich Über- und Unterdruck öfters abwechseln, so dass es mehrfach zu Positionsänderungen des Kolbens bzw. der Membran kommen kann. Um zu verhindern, dass das Zählwerk bei einem derartigen Druckverlauf während eines Sterilisationsprozesses mehrfach betätigt wird, wird mit Hilfe der Sperreinrichtung, insbesondere mittels des Bimetallstreifens die Steuerfunktion der Druckkammer außer Kraft gesetzt. Dies geschieht über die Steuerungsscheibe, die um eine Betätigung des Zählwerks zu verhindern, über eine Verformung des Bimetallstreifens relativpositioniert wird. Die Betätigungseinrichtung, insbesondere der Betätigungszapfen oder -stift des Zählwerkes steht nun in der oberen Stellung und wird durch die Steuerungsscheibe nach unten blockiert. Erwartet nun darauf, dass er wieder in die Ausgangsstellung gehen kann, um den Schaltvorgang zu beenden. Da die Temperatur während eines Sterilisationsprozesses normalerweise nicht unter 80°C fällt, reagiert das Bimetall erst nach Abschluss des Prozesses und kehrt in seine ursprüngliche Gestalt zurück, wenn zum Beispiel nahezu Raumtemperatur erreicht wird. In seiner ursprünglichen Gestalt führt das Bimetall die Steuerungsscheibe in ihre ursprüngliche Lage zurück, in der die Betätigungseinrichtung und insbesondere der Betätigungszapfen freigegeben ist. Wichtig ist, dass die Druckkammer nicht blockiert wird, sondern nach wie vor an jeweils vorliegende Druckverhältnisse und Druckunterschiede anpassen kann. Dies schont das gesamte System und macht es robust. Das Zählwerk reagiert quasi, obwohl es mechanisch ist, rein digital und kann insbesondere handelsüblich sein. Bei einem solchen Zählwerk wird durch einmaliges Drücken des Betätigungstiftes und loslassen die Zähleinheit um einen Zähler nach oben verschoben. Temperaturänderungen in der Sterilisationskammer steuern das Bimetall (durch beeinflussen seiner Gestalt oder Form), während Druckänderungen in der Kammer den pneumatischen Kolben steuern, also verlagern.

Weitere Merkmale und Vorteile der vorliegenden Erfindung ergeben sich aus der folgenden beispielhaften und nicht beschränkenden Beschreibung der Erfindung anhand von Figuren. Diese sind lediglich schematischer Natur und dienen nur dem Verständnis der Erfindung. Dabei zeigen:

Fig. 1 eine schematische Explosionsdarstellung einer Ausführungsform der Vorrichtung,

Fig. 2 eine schematische perspektivische Ansicht der Vorrichtung der Fig. 1,

Fign. 3a bis 3f jeweils freigeschnitten in perspektivischer Darstellung ausgewählte Komponenten der Vorrichtung in verschiedenen Funktionsstellungen,

Fign. 4a bis 4c die Vorrichtung im Schnitt zu verschiedenen Funktionsstellungen und

Fig. 5 ein Druck-Zeit-Diagramm als Beispiel für einen Sterilisationszyklus in einem fraktionierten Vakuumverfahren.

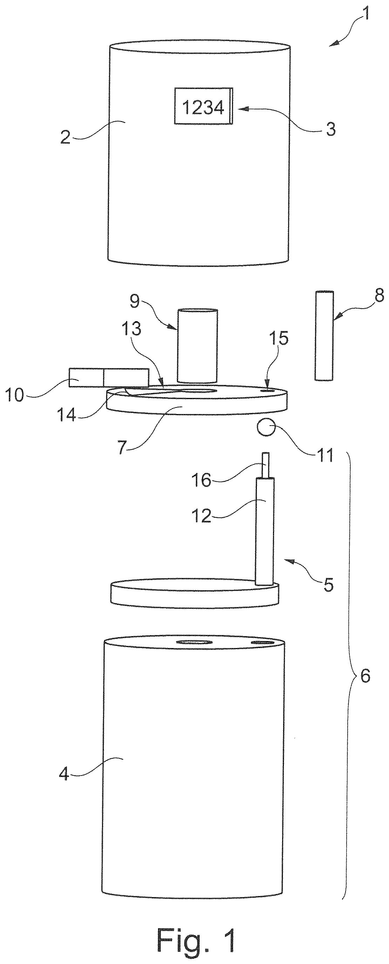

Figur 1 zeigt eine Ausführungsform der Vorrichtung 1 in einer perspektivischen Explosionsansicht. Diese weist ein mechanisches Zählwerk 2 mit einer Zählwerksanzeige 3, einen Zylinder 4, der zusammen mit einem Kolben 5 eine Kolben-Zylinder-Einheit als Betätigungseinrichtung 6 ausbildet, sowie eine Steuerscheibe 7 auf. Weitere Bestandteile der Vorrichtung sind ein Zählwerksstift 8, ein Führungsbolzen 9, über den die Steuerscheibe 7 verdrehbar am Zählwerk 2 gelagert ist, ein Bimetall 10, eine Kugel 11 als Kraftübertragungselement und einen Kolbenzapfen 12.

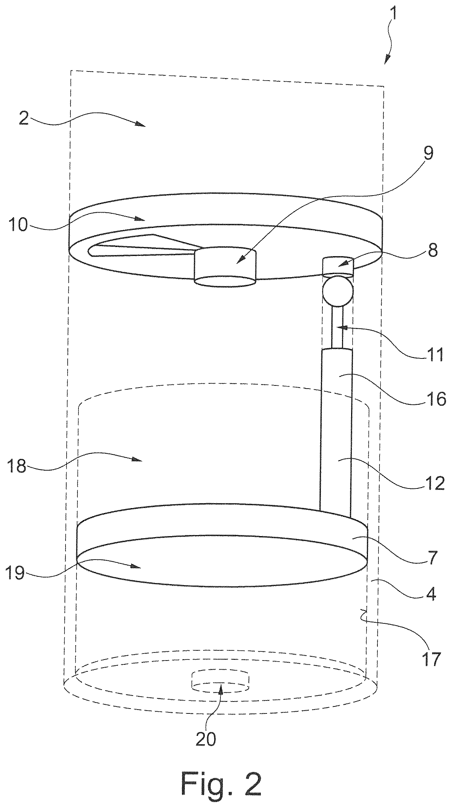

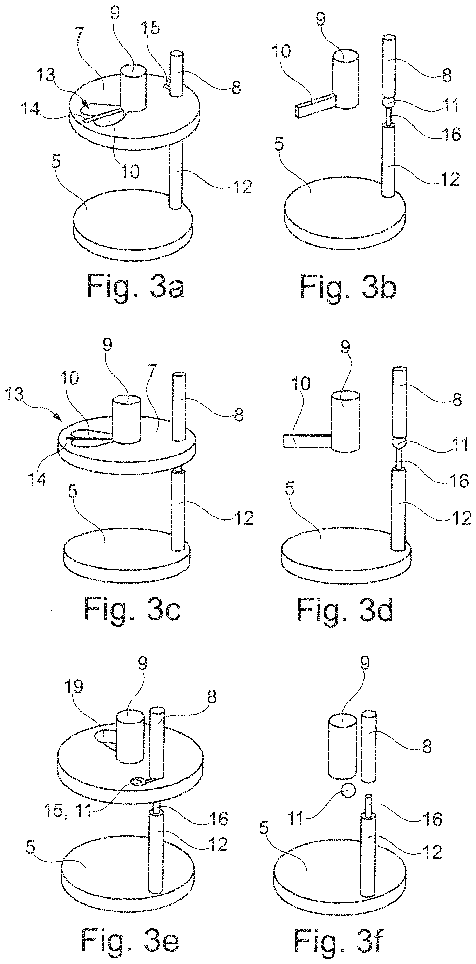

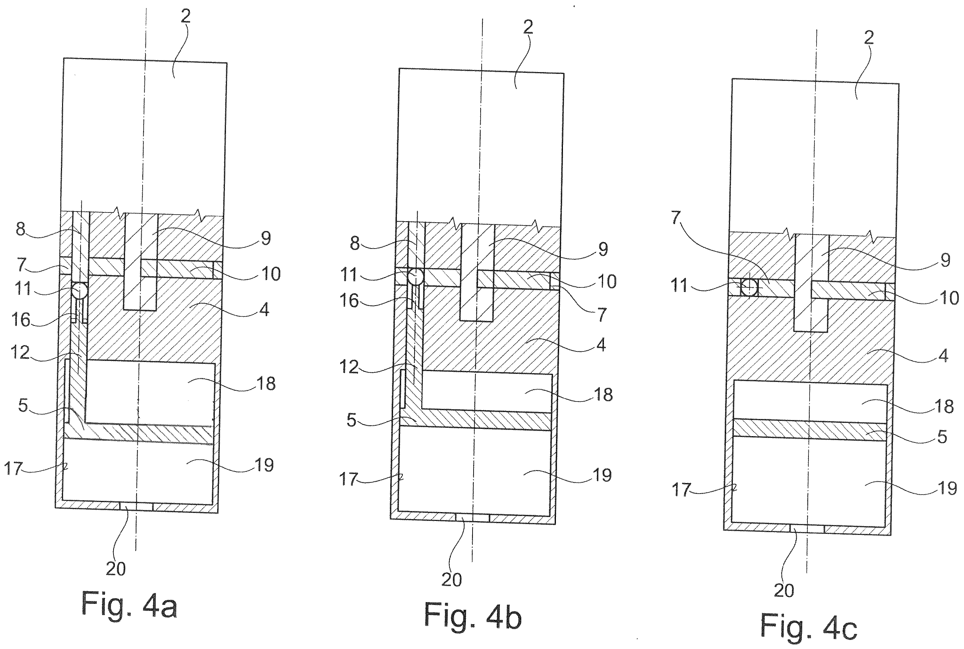

Die weitere Beschreibung erfolgt mit Bezugnahme auf die Figuren 1, 2, 3a bis 3f und 4. Das Zählwerk 2 weist eine in den Figuren nicht gezeigte zentrale Aufnahme auf, in der der Führungsbolzen 9 fixiert aufgenommen ist, z.B. eingeklebt ist. In den Führungsbolzen ist seitlich eine Ausnehmung eingebracht (siehe zum Beispiel Fign. 4a bis 4c), in der das Bimetallelement 10 derart befestigt ist, dass es in radialer Richtung vom Führungsbolzen 9 absteht. Die Steuerscheibe 7 weist radial außerhalb ihrer zentralen Ausnehmung eine Aussparung 13 als Aufnahme für das Bimetallelement 10 auf. Die Aussparung 13 ist, wie in Figur 3a angedeutet ist, in etwa herzförmig geformt, so dass sie in einem radial äußeren Bereich einen Klemmschlitz 14 für das Bimetallelement 10 besitzt. Insbesondere die Figuren 4a bis 4c zeigen, dass in axialer Richtung betrachtet das Bimetallelement 10 und die Scheibe 7 auf gleicher Höhe auf dem Führungsbolzen angeordnet sind.

Die Steuerscheibe 7 weist des Weiteren eine in den Figuren 3e und 4a bis 4c erkennbare Durchgangsöffnung 15 auf. Durch diese ragen je nach Funktionsstellung der Vorrichtung die Kugel 11, der Zählwerksstift 8 oder der Kolbenzapfen 12 hindurch. Die Durchgangsöffnung 15 ist näherungsweise schlüssellochförmig ausgebildet, mit einem weiten Abschnitt mit einem Durchmesser, der leicht weiter ist als die jeweiligen Durchmesser von Kugel 11 bzw. Zählwerksstift 8 bzw. Kolbenzapfen 12, durch den diese Elemente hindurchragen, und mit einem sich tangential daran anschließenden Abschnitt mit geringer Weite. Dessen Weite ist geringer als die Weite des Kolbenzapfens 12, jedoch weiter als die eines endseitig am Kolbenzapfen 12 ausgebildeten Kolbenstifts 16.

Der Kolben 5 ist mit den daran einstückig ausgebildeten Kolbenzapfen 12 nebst Kolbenstift 16 in dem Zylinder 4 aufgenommen (siehe Figuren 4a bis 4c). Er liegt dichtend an einer Innenwand 17 des Zylinders 4 an und kann sich in diesem in axialer Richtung bewegen. Zwischen dem Kolben 5 und dem Zylinder 4 sind einerseits eine Gegendruckkammer 18 und andererseits eine Sterilisationsdruckkammer 19 ausgebildet. Die Gegendruckkammer 18 ist durch den dichtenden Kontakt zwischen Kolben 5 und Zylinder 4 hermetisch geschlossen. Die Sterilisationsdruckkammer 19 hingegen ist mit einer Öffnung 20 versehen, die eine Verbindung zur, die Vorrichtung umgebenden Atmosphäre bewirkt. Über diese Öffnung 20 wirkt in der Sterilisationskammer 19 stets der die Vorrichtung 1 umgebende Außendruck, was im Falle eine Sterilisation der jeweilige Sterilisationsdruck ist.

Die Figuren 3a bis 3e zeigen den Kolben 5 mit Zählwerksstift 8, Kolbenzapfen 12, Kolbenstift 16, Scheibe 7, Bimetall 10, Kugel 11 und Führungsbolzen 9 in jeweils freigeschnittenen perspektivischen Darstellungen, wobei in den Figuren 3b, 3d und 3f zum Zwecke einer besseren Übersicht die Steuerscheibe 7 nicht dargestellt ist. Zu beachten ist, dass die Figuren die genannten Elemente aus leicht unterschiedlichen Perspektiven zeigen.

Die Figuren 3a und 3b sowie 4a zeigen einen ersten Betriebszustand, bei dem die Temperatur in einer nicht dargestellten Sterilisationskammer etwa 22°C, der Gegendruck in der Gegendruckkammer 18 ca. 1 bar und der Sterilisationsdruck in der Sterilisationsdruckkammer ebenfalls ca. 1 bar betragen. Dieser Zustand liegt zu Beginn eines Sterilisationszyklus vor. Das Bimetallelement 10 liegt bei diesen Parametern in seiner ursprünglichen Gestalt vor (zweite Gestalt). Die Durchgangsöffnung 15 befindet sich in Überdeckung mit Kolbenzapfen 12, Kolbenstift 16 und Kugel 11. Wie in Figur 4a gezeigt ist, ragt der nach unten in Richtung der Druckkammern 18, 19 federvorgespannte Zählwerksstift 8 durch die Durchgangsöffnung 15 hindurch und steht über die Kugel 11 mit dem Kolbenstift 16 und dem Kolbenzapfen 12 in Anlage.

Die Figuren 3c und 3d sowie 4b zeigen einen zweiten Betriebszustand, bei dem die Temperatur in einer nicht dargestellten Sterilisationskammer größer als 22°C, der Gegendruck in der Gegendruckkammer 18 größer als 1 bar und der Sterilisationsdruck in der Sterilisationsdruckkammer ebenfalls größer als 1 bar sind. In diesem Zustand hat sich der Druck (Sterilisationsdruck) im Druckzylinder 19 bzw. in der Sterilisationskammer gegenüber seinem ursprünglichen Wert und gegenüber dem Druck in der Gegendruckkammer 18 erhöht. Der pneumatische Kolben 5 bewegt sich infolge des Druckunterschieds nach oben in Richtung der Gegendruckkammer 18, so dass das darin eingeschlossene Gas komprimiert wird, bis näherungsweise ein Druckausgleich erreicht ist. Genauer gesagt gilt für das Druckgleichgewicht:

$$P_{Sterilisationskammer} = P_{Gegendruckkammer} + F_{Zählwerksstiftfeder} + Reibung$$

Der Kolbenzapfen 12 drückt über den Kolbenstift 16 und die Kugel 11 den federbelasteten Zählwerksstift 8 in Richtung des Zählwerks nach oben, wodurch ein Zählschritt eingeleitet wird. Die Steuerungsscheibe 7 ist nun vom Zählwerksstift 8 freigegeben und kann sich um den Führungsbolzen 9 verdrehen. Sobald der Zählwerksstift 8 die Scheibe 7 freigibt, befindet sich die Kugel 11 in der Durchgangsöffnung 15 und wird bei einer Rotation der Scheibe 7 um den Führungsbolzen 9 herum von der Scheibe 7 mitgenommen (siehe zum Beispiel in Figuren 3d und 3e). Da der Kolbenstift 16 eine Weite aufweist, die geringer als die Weite des engen Abschnitts der schlüssellochförmigen Durchgangsöffnung 15, ist eine Rotation der Scheibe 7 sowie eine Axialbewegung des Kolbens 5 mit Kolbenstift 16 ohne Konflikte möglich. Die Figuren 3c und 3d sowie 4b zeigen die genannten Elemente der Vorrichtung 1 in einem in axialer Richtung verschobenen, aber nicht radial verdrehten Zustand.

Die Figuren 3e und 3f sowie 4c zeigen einen dritten Betriebszustand, bei dem die Temperatur in einer nicht dargestellten Sterilisationskammer etwa zwischen 60°C und 134°C liegt und der Gegendruck in der Gegendruckkammer 18 ca. 0,2 bar bis 3 bar (absoluter Druck) und der Sterilisationsdruck in der Sterilisationsdruckkammer ebenfalls ca. 0,2 bar bis 3 bar betragen. Infolge der deutlich erhöhten Temperatur verformt sich das Bimetall 10 und übt auf die Steuerungsscheibe 7 ein Drehmoment aus. Infolgedessen verdreht sich die Steuerscheibe 7 in radialer Richtung, wobei die Kugel 11 vom Kolbenstift 16 und vom Zählwerkszapfen 8 getrennt wird, diese Elemente nicht mehr in gegenseitiger Anlage stehen und keine Kraft mehr in axialer Richtung übertragen werden kann, unabhängig von der Stellung des Kolbens 5. Auf diese Weise wird die Krafteinleitung von der Betätigungseinheit 6 in das Zählwerk 2 entkoppelt. Der Kolbenstift 16 am pneumatischen Kolben 5 hat am distalen Ende einen kleineren Durchmesser, so dass er in die Nut der Steuerungsscheibe 7 eintauchen kann, ohne den Zählwerksstift 8 zu berühren. Der Zählwerksstift 8 wird durch die Steuerungsscheibe 7 in Position gehalten, bis die Temperatur wieder bei Raumtemperatur angelangt ist und das Bimetall 10 die Steuerungsscheibe 7 zurück in die in den Figuren 3a bis 3d sowie 4a und 4b gezeigte Ausgangsposition verschiebt.

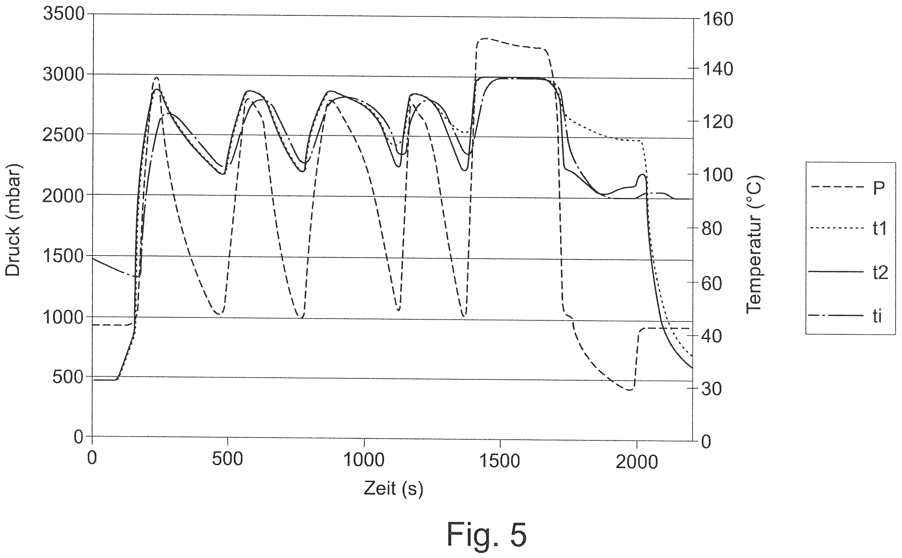

Die Verläufe der Parameterwerte von Temperatur und Druck sind für die vorstehend beschriebenen Vorgänge beispielhaft in Figur 5 gezeigt.

Bezugszeichenliste

1    Vorrichtung
2    Zählwerk
3    Zählwerksanzeige
4    Zylinder
5    Kolben
6    Betätigungseinrichtung
7    Steuerscheibe
8    Zählwerksstift
9    Führungsbolzen
10   Bimetallelement
11   Kugel, Kraftübertragungselement
12   Kolbenzapfen
13   Aussparung
14   Klemmschlitz
15   Durchgangsöffnung
16   Kolbenstift
17   Innenwand
18   Gegendruckkammer
19   Sterilisationsdruckkammer
20   Öffnung

Patentansprüche

1.   Vorrichtung (1) zum Zählen von Sterilisationszyklen bei der Sterilisierung medizinischer Instrumente und Vorrichtungen, aufweisend

ein Zählwerk (2) zum Aufzeichnen und Wiedergeben einer Anzahl der Sterilisationszyklen,

eine Betätigungseinrichtung (5, 6, 11, 12, 16) zur sterilisationsparameterabhängigen Betätigung des Zählwerks (2),

sowie eine Sperreinrichtung (7) zum sterilisationsparameterabhängigen Sperren des Zählwerks (2),

wobei die Betätigungseinrichtung (5, 6, 11, 12, 16) bei Überschreiten eines ersten Schwellsterilisationsdrucks das Zählwerk (2) betätigt und

die Sperreinrichtung (7) das Zählwerk (2) nach einer erfolgten Betätigung und/oder nach Überschreiten einer ersten Schwellsterilisationstemperatur sperrt und nach Unterschreiten einer zweiten Schwellsterilisationstemperatur entsperrt.

2. Vorrichtung (1) nach Anspruch 1, **dadurch gekennzeichnet, dass** die Betätigungseinrichtung (5, 6, 11, 12, 16) eine Kolben-Zylinder-Einheit (4, 5) mit einer vom Sterilisationsdruck beaufschlagbaren Kammer (18, 19) aufweist, wobei der Kolben (5) und der Zylinder (4) in Abhängigkeit vom Sterilisationsdruck zur Betätigung des Zählwerks (2) zueinander relativpositionierbar sind.

3. Vorrichtung (1) nach Anspruch 2, **dadurch gekennzeichnet, dass** die Sperreinrichtung (7) eine gegenüber der Kolben-Zylinder-Einheit (4, 5) bewegbare, insbesondere rotierbare Scheibe (7), mit einem Durchlass für das Betätigungselement (11, 12, 16) aufweist.

4. Vorrichtung (1) nach einem der vorstehenden Ansprüche, **dadurch gekennzeichnet, dass** die Sperreinrichtung (7) ein Bimetallelement (10) aufweist, das sich in Abhängigkeit der Sterilisationstemperatur zwischen einer ersten Gestalt und einer zweiten Gestalt verformt und in der ersten Gestalt das Zählwerk (2) von der Betätigungseinrichtung (5, 6, 11, 12, 16) entkoppelt und in der zweiten Gestalt das Zählwerk (2) mit der Betätigungseinrichtung (5, 6, 11, 12, 16) koppelt.

5. Vorrichtung nach Anspruch 3 und 4, **dadurch gekennzeichnet, dass** die Scheibe (7) mit dem Bimetallelement (10) bewegungsgekoppelt ist und bei in der ersten Gestalt befindlichem Bimetallelement (10) eine erste Stellung und bei in der zweiten Gestalt befindlichem Bimetallelement (10) eine zweite Stellung einnimmt.

6. Vorrichtung (1) nach Anspruch 5, **dadurch gekennzeichnet, dass** die Betätigungseinrichtung (5, 6, 11, 12, 16) einen Zapfen (12, 16) zur Betätigung des Zählwerks (2) aufweist, und die Scheibe (7) mit einem Durchlass (15) für den Zapfen (12, 16) versehen ist, der bei in der ersten Stellung befindlicher Scheibe (7) in Überdeckung mit dem Zapfen (12, 16) positioniert ist und eine Betätigung des Zählwerks (2) ermöglicht und bei in der zweiten Stellung befindlicher Scheibe (7) den Zapfen (12, 16) sperrt und eine Betätigung des Zählwerks (2) verhindert.

7. Vorrichtung (1) nach einem der Ansprüche 3 bis 6, **dadurch gekennzeichnet, dass** zwischen der Betätigungseinrichtung (5, 6, 11, 12, 16) und dem Zählwerk (2) in dem Durchlass (15) der Scheibe (7) ein Kraftübertragungselement (11), vorzugsweise eine Rolle oder eine Kugel (11), angeordnet ist.

8. Vorrichtung (1) nach einem der Ansprüche 3 bis 7, **dadurch gekennzeichnet, dass** der Durchlass (15) in der Scheibe (7) einen ersten Abschnitt mit einer ersten Weite und einen zweiten Abschnitt mit einer gegenüber dem ersten Abschnitt kleineren Weite aufweist.

9. Vorrichtung (1) nach Anspruch 7 und 8, **dadurch gekennzeichnet, dass** das Kraftübertragungselement (11) in dem ersten Abschnitt angeordnet ist und die Weite des Kraftübertragungselements (11) größer ist als die Weite des zweiten Abschnitts.

10. Vorrichtung (1) nach Anspruch 9, **dadurch gekennzeichnet, dass** der Zapfen (12) eine Weite aufweist, die größer als die Weite des ersten Abschnitts ist, und an seinem dem Kraftübertragungselement zugewandten Endabschnitt (16) eine verringerte Weite aufweist, die kleiner als die Weite des zweiten Abschnitts ist.

11. Vorrichtung (1) nach einem der Ansprüche 3 bis 10, **dadurch gekennzeichnet, dass** die Scheibe (7) rotierbar auf einem Führungsbolzen (9) aufgenommen ist, insbesondere eine zentrale Öffnung, insbesondere Durchgangsöffnung, aufweist, in der der Führungsbolzen (9) relativ zur Scheibe (7) rotierbar aufgenommen ist.

12. Vorrichtung (1) nach Anspruch 11, **dadurch gekennzeichnet, dass** das Bimetallelement (10) mit einem Endbereich an dem Führungsbolzen (9) angeordnet ist und mit einem diesem gegenüberliegenden Endbereich mit der Scheibe (7) verbunden ist.

**13.** Vorrichtung (1) nach Anspruch 3, **dadurch gekennzeichnet, dass** der Zylinder (4) ein Zylindergehäuse aufweist, in dem der Kolben (5) axialpositionierbar aufgenommen ist, und in dem die Scheibe (7) rotierbar aufgenommen ist, wobei endseitig an oder in dem Zylindergehäuse das Zählwerk (2) aufgenommen ist.

**Claims**

**1.** A device (1) for counting sterilization cycles during the sterilization of medical instruments and devices, comprising a counter (2) for recording and reproducing a number of the sterilization cycles,
an actuation unit (5, 6, 11, 12, 16) for a sterilization parameter-dependent actuation of the counter (2),
and a locking unit (7) for a sterilization parameter-dependent locking of the counter (2),
wherein the actuation unit (5, 6, 11, 12, 16) actuates the counter (2) upon exceeding a first threshold sterilization pressure and
the locking unit (7) locks the counter (2) after an actuation has been carried out and/or after exceeding a first threshold sterilization temperature, and unlocks it after falling below a second threshold sterilization temperature.

**2.** The device (1) according to claim 1, **characterized in that** the actuation unit (5, 6, 11, 12, 16) comprises a piston/cylinder unit (4, 5) having a chamber (18, 19) that can be acted upon by the sterilization pressure, the piston (5) and the cylinder (4), for actuating the counter (2), being capable of being positioned relative to each other depending on the sterilization pressure.

**3.** The device (1) according to claim 2, **characterized in that** the locking unit (7) comprises a disc (7) which can be moved, in particular rotated with respect to the piston/cylinder unit (4, 5) and has a passage for the actuation element (11, 12, 16).

**4.** The device (1) according to any of the preceding claims, **characterized in that** the locking unit (7) comprises a bimetal element (10) which is deformed depending on the sterilization temperature between a first shape and a second shape and decouples the counter (2) from the actuation unit (5, 6, 11, 12, 16) in the first shape and couples the counter (2) to the actuation unit (5, 6, 11, 12, 16) in the second shape.

**5.** The device according to claims 3 and 4, **characterized in that** the disc (7) is kinetically coupled to the bimetal element (10) and adopts a first position when the bimetal element (10) is in the first shape and adopts a second position when the bimetal element (10) is in the second shape.

**6.** The device (1) according to claim 5, **characterized in that** the actuation unit (5, 6, 11, 12, 16) comprises a stud/pin (12, 16) for actuating the counter (2) and the disc (7) is provided with a passage (15) for the stud/pin (12, 16), said passage being positioned so as to be in alignment with the stud/pin (12, 16) and allowing an actuation of the counter (2) when the disc (7) is in the first position and locks the stud/pin (12, 16) and prevents the counter (2) from being activated when the disc (7) is in the second position.

**7.** The device (1) according to any of the claims 3 to 6, **characterized in that** a force transmission element (11), preferably a roller or a ball (11), is arranged between the actuation unit (5, 6, 11, 12, 16) and the counter (2) in the passage (15) of the disc (7).

**8.** The device (1) according to any of the claims 3 to 7, **characterized in that** the passage (15) in the disc (7) comprises a first portion having a first width and a second portion having a width smaller than that of the first portion.

**9.** The device (1) according to claims 7 and 8, **characterized in that** the force transmission element (11) is arranged in the first portion and the width of the force transmission element (11) is larger than that of the second portion.

**10.** The device (1) according to claim 9, **characterized in that** the stud (12) has a width which is larger than that of the first portion, and its end portion (16) facing the force transmission element has a reduced width which is smaller than that of the second portion.

**11.** The device (1) according to any of the claims 3 to 10, **characterized in that** the disc (7) is rotatably supported on a guide bolt (9) and comprises in particular a central opening, especially a through-hole in which the guide bolt (9) is received to be rotatable relative to the disc (7).

**12.** The device (1) according to claim 11, **characterized in that** one end section of the bimetal element (10) is arranged on the guide bolt (9) and the other, opposite end section is connected to the disc (7).

**13.** The device (1) according to claim 3, **characterized in that** the cylinder (4) comprises a cylinder housing in which the piston (5) is received to be axially positionable, and in which the disc (7) is rotatably received, with the counter (2) being received at the end side on or in the cylinder housing.

**Revendications**

**1.** Dispositif (1) de comptage de cycles de stérilisation lors de la stérilisation d'instruments et de dispositifs médicaux, présentant
un compteur (2) destiné à enregistrer et à restituer un nombre de cycles de stérilisation,
un dispositif d'activation (5, 6, 11, 12, 16) destiné à activer le compteur (2) en fonction d'un paramètre de stérilisation,
et un dispositif de blocage (7) destiné à bloquer le compteur (2) en fonction d'un paramètre de stérilisation,
dans lequel le dispositif d'activation (5, 6, 11, 12, 16) active le compteur (2) en cas de dépassement d'une première pression de stérilisation seuil, et
le dispositif de blocage (7) bloque le compteur (2) après une activation effectuée et/ou après dépassement d'une première température de stérilisation seuil et le débloque après passage en-dessous d'une seconde température de stérilisation seuil.

**2.** Dispositif (1) selon la revendication 1, **caractérisé en ce que** le dispositif d'activation (5, 6, 11, 12, 16) présente une unité piston-cylindre (4, 5) avec une chambre (18, 19) pouvant être soumise à une pression de stérilisation, dans lequel le piston (5) et le cylindre (4) peuvent être positionnés l'un par rapport à l'autre en fonction de la pression de stérilisation pour activer le compteur (2).

**3.** Dispositif (1) selon la revendication 2, **caractérisé en ce que** le dispositif de blocage (7) présente un disque en particulier rotatif (7) et mobile par rapport à l'unité piston-cylindre (4, 5), avec un passage pour l'élément d'activation (11, 12, 16).

**4.** Dispositif (1) selon l'une des revendications précédentes, **caractérisé en ce que** le dispositif de blocage (7) présente un élément bimétallique (10), qui se déforme en fonction de la température de stérilisation entre une première forme et une seconde forme, et qui dans la première forme découple le compteur (2) du dispositif d'activation (5, 6, 11, 12, 16), et dans la seconde forme couple le compteur (2) au dispositif d'activation (5, 6, 11, 12, 16).

**5.** Dispositif selon les revendications 3 et 4, **caractérisé en ce que** le disque (7) est couplé en déplacement avec l'élément bimétallique (10), et occupe une première position lorsque l'élément bimétallique (10) est dans la première forme, et occupe une seconde position lorsque l'élément bimétallique (10) est dans la seconde forme.

**6.** Dispositif (1) selon la revendication 5, **caractérisé en ce que** le dispositif d'activation (5, 6, 11, 12, 16) présente une broche (12, 16) destinée à activer le compteur (2), et le disque (7) est prévu avec un passage (15) pour la broche (12, 16) qui, lorsque le disque (7) se trouve dans la première position, est positionné coïncidant avec la broche (12, 16) et permet une activation du compteur (2), et lorsque le disque (7) se trouve dans la seconde position, bloque la broche (12, 16) et empêche une activation du compteur (2).

**7.** Dispositif (1) selon l'une quelconque des revendications 3 à 6, **caractérisé en ce qu'**entre le dispositif d'activation (5, 6, 11, 12, 16) et le compteur (2), dans le passage (15) du disque (7), est agencé un élément de transmission de force (11), de préférence un rouleau ou une bille (11).

**8.** Dispositif (1) selon l'une quelconque des revendications 3 à 7, **caractérisé en ce que** le passage (15) dans le disque (7) présente une première section avec une première largeur et une seconde section avec une largeur inférieure à la première section.

**9.** Dispositif (1) selon les revendications 7 et 8, **caractérisé en ce que** l'élément de transmission de force (11) est agencé dans la première section et la largeur de l'élément de transmission de force (11) est supérieure à la largeur de la seconde section.

**10.** Dispositif (1) selon la revendication 9, **caractérisé en ce que** la broche (12) présente une largeur, qui est supérieure à la largeur de la première section, et au niveau de sa section d'extrémité (16) tournée vers l'élément de transmission de force, présente une largeur réduite, qui est inférieure à la largeur de la seconde section.

**11.** Dispositif (1) selon l'une quelconque des revendications 3 à 10, **caractérisé en ce que** le disque (7) est logé de manière rotative sur un boulon de guidage (9), en particulier présente une ouverture centrale, en particulier une ouverture traversante, dans laquelle le boulon de guidage (9) est logé de manière rotative par rapport au disque (7).

**12.** Dispositif (1) selon la revendication 11, **caractérisé en ce que** l'élément bimétallique (10) est agencé avec une région d'extrémité au niveau du boulon de guidage (9), et est relié au disque (7) à une région d'extrémité opposée à celle-ci.

**13.** Dispositif (1) selon la revendication 3, **caractérisé en ce que** le cylindre (4) présente un logement de cylindre, dans lequel le piston (5) est logé de manière à pouvoir être positionné axialement et dans lequel le disque (7) est logé de manière rotative, dans lequel le compteur (2) est logé en extrémité sur ou dans le logement de cylindre.

1234

Fig. 1

Fig. 2

Fig. 3a

Fig. 3b

Fig. 3c

Fig. 3d

Fig. 3e

Fig. 3f

Fig. 4a    Fig. 4b    Fig. 4c

EP 3 328 446 B1

EP 3 328 446 B1

Fig. 5

**EP 3 328 446 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0979658 A1
- DE 102007039088 A1
- US 5359993 A
- US 2014000506 A1